# EUROPEAN PATENT APPLICATION

(11) **EP 4 588 475 A1**
(43) Date of publication of application: **23.07.2025**
(21) Application number: 23864716.8
(22) Date of filing: 13.09.2023
(51) Int. Cl.: A61K 31/4422, A61K 31/353, A61P 9/12, A61P 9/04, A61P 9/00

(54) **NEBIVOLOL AND AMLODIPINE COMPOSITION, PREPARATION METHOD THEREFOR, AND USE THEREOF**

(30) Priority: 14.09.2022 CN 202211112943
(71) Applicant: Shanghai Yonsun Biotechnology Co., Ltd., Shanghai 201210 (CN)
(72) Inventor: GUO, Zhen, Shanghai 201210 (CN); XIE, Wenfeng, Shanghai 201210 (CN); CHEN, Zuyou, Shanghai 201210 (CN); WANG, Tingting, Shanghai 201210 (CN); YING, Shuhuan, Shanghai 201210 (CN)
(74) Representative: Gille Hrabal Partnerschaftsgesellschaft mbB Patentanwälte
(86) International application number: PCT/CN2023/118513
(87) International publication number: WO 2024/055984

(57) **Abstract**

Provided is a nebivolol and amlodipine composition, which comprises nebivolol and amlodipine. The nebivolol comprises one or more of nebivolol, a pharmaceutically acceptable salt thereof, and a solvate thereof. The amlodipine comprises one or more of amlodipine, a pharmaceutically acceptable salt thereof, and a solvate thereof. A nebivolol and amlodipine compound preparation is beneficial to improving the blood pressure reduction effect, improving the safety and tolerance, and reducing side effects. Compared with single medicines of nebivolol and amlodipine, the combination of nebivolol and amlodipine has the advantages that: the number of medicines taken is reduced, and the compliance of a patient is improved; the medicine-taking compliance of old people or people with dysphagia can be facilitated; a plasma concentration in vivo is maintained to be stable, the blood pressure of a hypertension patient is maintained to be stable for a long time, and the side effects of the single medicines are reduced; and it is avoided that a patient stops taking medicine at will, and disease recurrence and progression of malignant complications are prevented.

## Description

The present application claims priority to the prior application with the patent application No. 202211112943.4, entitled "NEBIVOLOL AND AMLODIPINE COMPOSITION, PREPARATION METHOD THEREFOR, AND USE THEREOF" and filed with the China National Intellectual Property Administration by the applicant on September 14, 2022, which is incorporated herein by reference in its entirety.

### TECHNICAL FIELD

The present disclosure relates to a nebivolol and amlodipine composition, a preparation method therefor, and use thereof.

### BACKGROUND

Hypertension is one of the most common cardiovascular diseases, affecting 25%-30% of the adult population. If left uncontrolled over the long run, hypertension can increase the risk of developing coronary atherosclerosis, left ventricular hypertrophy, carotid atherosclerosis, and kidney disease. Currently, the diagnosis and treatment of hypertension mainly involve pharmaceutical interventions, and they have demonstrated significant clinical effects and can effectively inhibit the disease and alleviate patients' clinical symptoms. Therefore, the development of pharmaceutical formulations for the treatment of hypertension and cardiovascular diseases with reduced toxic and side effects is of positive significance for clinical medication.

The chemical name of nebivolol is 2,2'-[iminobis(methylene)]bis[6-fluoro-3,4-dihydro-2H-1-benzopyran-2-methanol]bis[2-hydroxy-2-(6fluoro-3,4-dihydro-2H-1-benzopyran-2-)]ethylamine, its molecular formula is C₂₂H₂₅F₂NO₄, its molecular weight is 405.43, and its structural formula is as follows:

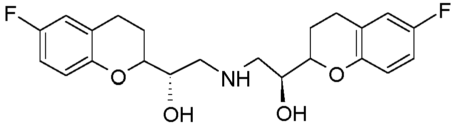

Nebivolol, as a potent and highly selective (cardioselective) β1 adrenergic receptor blocker with vasodilatory activity, has no α-adrenergic antagonistic effect. Due to its vasodilatory effect, it has a certain protective effect on heart function: it can reduce cardiac preload. Nebivolol is mainly used for the treatment of essential hypertension and chronic heart failure, with indications including hypertension, angina, myocardial infarction, and congestive heart failure, among other cardiovascular diseases. Nebivolol hydrochloride tablets have advantages such as having significant efficacy, being convenient to take, having few adverse effects, etc., making them a new promising antihypertensive drug. They were approved for the treatment of essential hypertension for the first time in Germany in 1997. However, common adverse effects of β receptor blockers include headache, nausea, bradycardia, orthostatic hypotension, bronchospasm, etc.

The chemical name of amlodipine is ethyl methyl 6-methyl-2-(2-aminoethoxy)methyl-4-(2-chlorophenyl)-1,4-dihydro-3,5-pyridinedicarboxylate, its molecular formula is C₂₀H₂₅CIN₂O₅, its molecular weight is 408.88, and its structural formula is as follows:

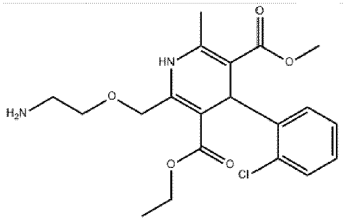

Amlodipine is a dihydropyridine calcium ion influx inhibitor (slow channel blocker or calcium ion antagonist) that inhibits the transmembrane influx of calcium ions into cardiac and vascular smooth muscles. The mechanism of action of amlodipine in lowering blood pressure is its direct relaxant effect on vascular smooth muscle. While the exact mechanism by which amlodipine alleviates angina is not fully determined, amlodipine is known to reduce overall ischemic burden through two effects: 1. Amlodipine causes dilation of peripheral arterioles, thereby reducing the total peripheral resistance (afterload) against which the heart works. Since the heart rate remains stable, this unloading in the heart reduces myocardial energy consumption and oxygen demand. 2. The mechanism of action of amlodipine may also involve dilation of the main coronary arteries and coronary arterioles in normal and ischemic regions. This dilation increases myocardial oxygen delivery in patients with coronary artery spasm (Prinzmetal's or variant angina). Amlodipine is a long-acting calcium channel blocker used for the treatment of hypertension, chronic stable angina, and vasospastic angina.

However, the prior art is limited to monotherapy formulations. Therefore, there is an urgent need to develop a novel compound formulation with good synergy, good stability, few side effects, good antihypertensive efficacy, and good patient compliance.

### SUMMARY

The technical problem that the present disclosure solves is to provide a nebivolol and amlodipine composition that is different from the prior art, a preparation method therefor, and use thereof.

The present disclosure provides a nebivolol and amlodipine composition comprising an active ingredient nebivolol and an active ingredient amlodipine, wherein the active ingredient nebivolol comprises one or more of nebivolol, a pharmaceutically acceptable salt thereof, and a solvate thereof, and the active ingredient amlodipine comprises one or more of amlodipine, a pharmaceutically acceptable salt thereof, and a solvate thereof.

According to an embodiment of the present disclosure, the pharmaceutically acceptable salt of nebivolol can be nebivolol hydrochloride.

According to an embodiment of the present disclosure, the pharmaceutically acceptable salt of amlodipine can be amlodipine besylate and/or amlodipine maleate.

According to an embodiment of the present disclosure, the nebivolol and amlodipine composition can be a nebivolol hydrochloride and amlodipine besylate composition.

According to an embodiment of the present disclosure, the nebivolol and amlodipine composition further comprises one or more of a surfactant, a filler, an adhesive, a disintegrant, a lubricant, a glidant, a colorant, and a coating film.

According to an embodiment of the present disclosure, the surfactant can be sodium dodecyl sulfate and/or polysorbate; for example, the polysorbate can be selected from polysorbate-80 and/or polysorbate-20.

According to an embodiment of the present disclosure, the filler refers to a solid substance that is added to a material to improve the properties of the material, or to solubilize and increase weight, and to reduce the cost of the material and is selected from one or more of microcrystalline cellulose (e.g., microcrystalline cellulose 101 or microcrystalline cellulose 102), dicalcium phosphate, mannitol, sucrose, glucose, maltose, lactose (e.g., lactose monohydrate), sorbitol, xylitol, maltitol, galactitol, erythritol, dextrin, and trehalose.

According to an embodiment of the present disclosure, the adhesive can be a conventional additive in the art that is capable of increasing the viscosity of a dispersion medium to reduce the settling rate of microparticles or increase the hydrophilicity of microparticles and is selected from one or more of polyoxyethylene, hydroxypropyl methylcellulose, hydroxypropylcellulose, hydroxyethylcellulose, ethylcellulose, copovidone, povidone (e.g., povidone K30), pregelatinized starch, acacia, polyvinylpyrrolidone, and sodium alginate.

According to an embodiment of the present disclosure, the disintegrant refers to a substance that is capable of disintegrating a formulation (e.g., tablets or granules) prepared from the composition in dissolution and is selected from one or more of crospovidone, croscarmellose sodium, sodium carboxymethyl starch, and corn starch.

According to an embodiment of the present disclosure, the lubricant can be a conventional substance in the art that has a lubricative effect and is selected from one or more of a metal stearate, stearic acid, talc, a stearic acid ester, stearyl fumarate, and micronized silica gel. The metal stearate is preferably one or more of magnesium stearate, calcium stearate, and sodium stearyl fumarate. The stearic acid ester is preferably glyceryl stearate.

According to an embodiment of the present disclosure, the glidant can be a conventional auxiliary material in the art that is capable of reducing the friction between particles to improve the flowability of powders or granules and is selected from one or more of talc, micronized silica gel, and colloidal silicon dioxide.

According to an embodiment of the present disclosure, the colorant can be a conventional substance in the art that is capable of achieving coloring purposes and is selected from iron oxide red and/or iron oxide yellow.

According to an embodiment of the present disclosure, components in the nebivolol and amlodipine composition, other than the coating film, collectively constitute a core.

According to an embodiment of the present disclosure, the nebivolol, the pharmaceutically acceptable salt thereof, or the solvate thereof makes up 1-10%, e.g., 1.5-7%, illustratively 1.60%, 1.82%, 2.00%, 2.18%, 2.27%, 2.50%, 2.73%, 3.00%, 3.50%, 4.00%, 5.00%, 5.45%, or 6.00%, by mass of the core. According to an embodiment of the present disclosure, the amlodipine, the pharmaceutically acceptable salt thereof, or the solvate thereof makes up 1-20%, e.g., 1.5-15%, illustratively 2.00%, 2.31%, 2.77%, 2.89%, 3.00%, 4.00%, 5.00%, 5.45%, 6.00%, 6.93%, 8.00%, 10.00%, 12.00%, 13.86%, or 14.00%, by mass of the core.

According to an embodiment of the present disclosure, the surfactant makes up 0.05-10%, e.g., 0.1-5%, illustratively 0.19%, 0.25%, 0.38%, 0.50%, 1.20%, 2.00%, or 3.00%, by mass of the core. According to an embodiment of the present disclosure, the filler makes up 40-95%, e.g., 60-90%, illustratively 68.25%, 79.70%, 88.07%, 78.77%, 79.14%, 81.05%, 66.06%, 66.00%, or 65.81%, by mass of the core.

According to an embodiment of the present disclosure, the adhesive makes up 1-30%, e.g., 5-25%, illustratively 5.00%, 6.67%, 8.00%, 10.00%, 18.00%, or 20.80%, by mass of the core.

According to an embodiment of the present disclosure, the disintegrant makes up 1-10%, e.g., 2-8%, illustratively 4.00%, 5.00%, 6.00%, 6.60%, or 7.00%, by mass of the core.

According to an embodiment of the present disclosure, the lubricant makes up 0.1-5%, e.g., 0.5-3%, illustratively 1.00%, 1.50%, or 2.00%, by mass of the core.

According to an embodiment of the present disclosure, the glidant makes up 0.1-5%, e.g., 0.2-3%, illustratively 0.25%, 0.30%, 0.40%, 0.50%, 1.00%, or 2.00%, by mass of the core.

According to an embodiment of the present disclosure, the coating film of the present disclosure can be selected from a film coating premix (gastric-soluble).

According to an embodiment of the present disclosure, the core can be any one of the following formulas:
formula 1: 2.77% amlodipine besylate, 2.18% nebivolol hydrochloride, 44.25% microcrystalline cellulose 101, 16.00% lactose monohydrate, 12.00% pregelatinized starch, 6.00% sodium carboxymethyl starch, 6.00% hydroxypropylcellulose, 1.20% polysorbate-80, 8.00% microcrystalline cellulose 102, 0.60% colloidal silicon dioxide, and 1.00% magnesium stearate, wherein each of the percentages refers to a percentage of the corresponding component's mass in the core's total mass;
formula 2:
   2.31% amlodipine besylate, 1.82% nebivolol hydrochloride, 39.70% mannitol, 20.00% lactose monohydrate, 6.67% hydroxypropyl methylcellulose, 6.00% croscarmellose sodium, 2.00% sodium dodecyl sulfate, 10.00% microcrystalline cellulose 102, 10.00% anhydrous dicalcium phosphate, 0.50% colloidal silicon dioxide, and 1.00% magnesium stearate, wherein each of the percentages refers to a percentage of the corresponding component's mass in the core's total mass;
formula 3:
   an amlodipine layer: 6.93% amlodipine besylate, 60.00% microcrystalline cellulose 102, 28.07% lactose monohydrate, 4.00% sodium carboxymethyl starch, and 1.00% magnesium stearate, wherein each of the percentages refers to a percentage of the corresponding component's weight in the amlodipine layer's total mass; and
   a nebivolol layer: 2.73% nebivolol hydrochloride, 63.77% microcrystalline cellulose 101, 15.00% lactose monohydrate, 6.00% croscarmellose sodium, 8.00% pregelatinized starch, 3.00% polysorbate-80, 0.50% colloidal silicon dioxide, and 1.00% magnesium stearate, wherein each of the percentages refers to a percentage of the corresponding component's weight in the nebivolol layer's total mass;
formula 4:
   an amlodipine layer: 13.86% amlodipine besylate, 54.14% microcrystalline cellulose 102, 25.00% anhydrous dicalcium phosphate, 6.00% sodium carboxymethyl starch, and 1.00% magnesium stearate, wherein each of the percentages refers to a percentage of the corresponding component's weight in the amlodipine layer's total mass; and
   a nebivolol layer: 5.45% nebivolol hydrochloride, 56.05% microcrystalline cellulose 101, 25.00% mannitol, 5.00% croscarmellose sodium, 5.00% hydroxypropylcellulose, 2.00% polysorbate-80, 0.50% colloidal silicon dioxide, and 1.00% magnesium stearate, wherein each of the percentages refers to a percentage of the corresponding component's weight in the nebivolol layer's total mass;
formula 5: 2.27% nebivolol hydrochloride, 2.89% amlodipine besylate, 59.41% lactose monohydrate, 19.16% pregelatinized starch, 2.87% croscarmellose sodium (internal), 1.63% hydroxypropyl methylcellulose, 0.38% polysorbate-80, 0.25% sodium dodecyl sulfate, 3.74% croscarmellose sodium (external), 0.25% colloidal silicon dioxide, 6.65% microcrystalline cellulose 101, and 0.50% magnesium stearate, wherein each of the percentages refers to a percentage of the corresponding component's mass in the core's total mass;
formula 6: 2.27% nebivolol hydrochloride, 2.89% amlodipine besylate, 59.35% lactose monohydrate, 19.16% pregelatinized starch, 2.87% croscarmellose sodium (internal), 1.63% hydroxypropyl methylcellulose, 0.19% polysorbate-80, 3.74% croscarmellose sodium (external), 0.25% colloidal silicon dioxide, 6.65% microcrystalline cellulose 101, 0.50% sodium dodecyl sulfate, and 0.50% magnesium stearate, wherein each of the percentages refers to a percentage of the corresponding component's mass in the core's total mass;
formula 7: 2.27% nebivolol hydrochloride, 2.89% amlodipine besylate, 59.16% lactose monohydrate, 19.16% pregelatinized starch, 2.87% croscarmellose sodium (internal), 1.63% hydroxypropyl methylcellulose, 0.38% polysorbate-80, 0.50% sodium dodecyl sulfate, 3.74% croscarmellose sodium (external), 0.25% colloidal silicon dioxide, 6.65% microcrystalline cellulose 101, and 0.50% magnesium stearate, wherein each of the percentages refers to a percentage of the corresponding component's mass in the core's total mass; and
formula 8: 1.56% nebivolol hydrochloride, 1.98% amlodipine besylate, 22.85% microcrystalline cellulose (internal), 11.43% anhydrous dicalcium phosphate, 38.25% lactose monohydrate, 11.43% pregelatinized starch, 1.29% hydroxypropyl methylcellulose, 0.26% polysorbate-80, 0.17% sodium dodecyl sulfate, 4.28% microcrystalline cellulose (external), 5.00% croscarmellose sodium (external), 0.50% colloidal silicon dioxide, and 1.00% magnesium stearate, wherein each of the percentages refers to a percentage of the corresponding component's mass in the core's total mass.

According to an embodiment of the present disclosure, the nebivolol and amlodipine composition can comprise a nebivolol dose of 2.5 mg to 20.0 mg, e.g., 2.5 mg, 5.0 mg, 7.5 mg, 10.0 mg, 15.0 mg, or 20.0 mg.

According to an embodiment of the present disclosure, the nebivolol and amlodipine composition can comprise an amlodipine dose of 2.5 mg to 20.0 mg, e.g., 2.5 mg, 5.0 mg, 7.5 mg, 10.0 mg, 15.0 mg, or 20.0 mg.

According to an embodiment of the present disclosure, both nebivolol and amlodipine in the nebivolol and amlodipine composition have a dissolution amount of 70% or more, e.g., 75% or more, preferably 80% or more, within 30 min.

The present disclosure further provides use of the nebivolol and amlodipine composition for manufacturing a medicament for the treatment and/or prevention of cardiovascular diseases. According to an embodiment of the present disclosure, the cardiovascular diseases include hypertension, heart failure, coronary heart disease, angina, arrhythmia, myocardial infarction, congenital heart disease, heart valve disease, etc.

The present disclosure further provides a method of treatment and/or prevention of cardiovascular diseases, the method comprising administering to a mammal (e.g., a human) in need thereof a therapeutically effective amount of the nebivolol and amlodipine composition, the medicament, or the following pharmaceutical formulation.

The present disclosure further provides use of the nebivolol and amlodipine composition for manufacturing a pharmaceutical formulation.

According to an embodiment of the present disclosure, the pharmaceutical formulation can be an oral pharmaceutical formulation; for example, dosage forms of the oral pharmaceutical formulation include, but are not limited to, compound tablets, double-layer tablets, capsules, pellets, micro-tablets, etc.

The present disclosure further provides a pharmaceutical formulation prepared from the above nebivolol and amlodipine composition. Preferably, the pharmaceutical formulation has the definition described above.

The present disclosure further provides a preparation method for the nebivolol and amlodipine composition, the method comprising, but not limited to, a direct powder compression method, a wet granulation method, a dry granulation method, a fluidized granulation method, an extrusion-spheronization method, or a pellet coating method.

According to an embodiment of the present disclosure, the wet granulation method or the fluidized granulation method can comprise the following steps:
step 1: mixing nebivolol hydrochloride with the filler, the adhesive, and the disintegrant, then mixing with an aqueous adhesive solution, performing wet granulation, wet sizing, drying, and dry sizing to give dry granules of nebivolol;
step 2: mixing the dry granules of nebivolol obtained in step 1 with amlodipine and the filler, then mixing with the lubricant and the glidant, and tableting to give nebivolol-amlodipine plain tablets; and
step 3: coating the nebivolol-amlodipine plain tablets obtained in step 2 with an aqueous solution of a film coating premix to give nebivolol-amlodipine tablets.

According to an embodiment of the present disclosure, the dry granulation method can comprise the following steps:
step ①: mixing nebivolol hydrochloride with the filler, the adhesive, and the disintegrant and then performing, with an aqueous adhesive solution, granulation, wet sizing, drying, and dry sizing to give dry granules of nebivolol; and then mixing the obtained dry granules of nebivolol with the lubricant and the glidant to give final mixed granules of nebivolol;
step ②: homogeneously mixing amlodipine with the filler and the disintegrant; and then granulating by using a dry granulator and homogeneously mixing with the lubricant to give final mixed granules of amlodipine; and
step ③: tableting the final mixed granules of nebivolol obtained in step ① and the final mixed granules of amlodipine obtained in step ② to give nebivolol-amlodipine plain tablets; and then performing film coating to give nebivolol-amlodipine tablets.

The order of the above steps ① and ② can be changed.

The reagents and starting materials used in the present disclosure are commercially available.

### Beneficial Effects

The present disclosure provides a compound formulation of nebivolol and amlodipine used for the treatment and/or prevention of cardiovascular diseases.

### DETAILED DESCRIPTION

The technical solutions of the present disclosure will be further described in detail with reference to the following specific examples. It will be appreciated that the following examples are merely exemplary illustrations and explanations of the present disclosure and should not be construed as limiting the claimed scope of the present disclosure. All techniques implemented on the basis of the content described above of the present disclosure are encompassed within the claimed scope of the present disclosure.

Unless otherwise stated, the starting materials and reagents used in the following examples are all commercially available products or can be prepared by using known methods.

### Example 1

### 1) Formula composition

| Name | Amount per tablet (mg) | Percent (%) |
|---|---|---|
| **Core** | | |
| Amlodipine besylate | 6.93 | 2.77 |
| Nebivolol hydrochloride | 5.45 | 2.18 |
| Microcrystalline cellulose 101 | 110.62 | 44.25 |
| Lactose monohydrate | 40 | 16 |
| Pregelatinized starch | 30 | 12 |
| Sodium carboxymethyl starch | 15 | 6 |
| Hydroxypropyl methylcellulose | 15 | 6 |
| Polysorbate-80 | 3 | 1.2 |
| Microcrystalline cellulose 102 | 20 | 8 |
| Colloidal silicon dioxide | 1.5 | 0.6 |
| Magnesium stearate | 2.5 | 1 |

| **Coating material** | | |
|---|---|---|
| Film coating premix | 10 | / |

### 2) Preparation method

Adhesive preparation: An adhesive (5% solid content) was prepared from hydroxypropyl methylcellulose and purified water, and polysorbate-80 was then added. The mixture was stirred homogeneously and set aside for later use.

Step 1: Nebivolol hydrochloride, microcrystalline cellulose 101, lactose monohydrate, pregelatinized starch, and sodium carboxymethyl starch were placed into a wet granulation vessel. The stirring speed and cutting speed were activated, and the materials were mixed for 5 min. The aqueous adhesive solution was then added, and wet granulation was performed. The granulated material was wet-sized using a 16-mesh sieve. The sized material was placed into an oven and dried until the water content was less than 3.0%. The dried granules were passed through a 20-mesh sieve for dry sizing to give dry granules of nebivolol hydrochloride.

Step 2: The dry granules of nebivolol hydrochloride obtained in step 1, amlodipine besylate, and microcrystalline cellulose 102 were added to a mixing vessel and mixed for 20 min. Colloidal silicon dioxide and magnesium stearate were then added, and the materials were mixed for 5 min. The mixture was then tableted using a rotary tablet press to give nebivolol-amlodipine plain tablets. Step 3: The nebivolol-amlodipine plain tablets obtained in step 2 were coated with an aqueous solution of a film coating premix (12% solid content; the percentage refers to the percentage of the mass of the coating premix in the total mass of the aqueous solution of the coating premix) to give nebivolol-amlodipine tablets.

### Example 2

### 1) Formula composition

| Name | Amount per tablet (mg) | Percent (%) |
|---|---|---|
| **Core** | | |
| Amlodipine besylate | 6.93 | 2.31 |
| Nebivolol hydrochloride | 5.45 | 1.82 |
| Mannitol | 119.12 | 39.70 |
| Lactose monohydrate | 60 | 20.00 |
| Hydroxypropyl methylcellulose | 20 | 6.67 |
| Croscarmellose sodium | 18 | 6.00 |
| Sodium dodecyl sulfate | 6 | 2.00 |
| Microcrystalline cellulose 102 | 30 | 10.00 |
| Anhydrous dicalcium phosphate | 30 | 10.00 |
| Colloidal silicon dioxide | 1.5 | 0.50 |
| Magnesium stearate | 3 | 1.00 |

| Coating material | | |
|---|---|---|
| Film coating premix | 9 | / |

### 2) Preparation method

Adhesive preparation: An adhesive (5% solid content) was prepared from hydroxypropyl methylcellulose and purified water, and sodium dodecyl sulfate was then added and dissolved by stirring. The resulting solution was set aside for later use.

Step 1: Nebivolol hydrochloride, mannitol, lactose monohydrate, and croscarmellose sodium were added to a mixing vessel and mixed for 20 min. The mixed material was then placed into a fluidized bed, and the inlet airflow rate, inlet air temperature, atomization temperature, and feeding rate parameters were set. The aqueous adhesive solution was added using a peristaltic pump, and fluidized granulation was performed. The granulated material was passed through a 30-mesh sieve to give dry granules of nebivolol.

Step 2: The dry granules of nebivolol obtained in step 1, amlodipine besylate, microcrystalline cellulose 102, anhydrous dicalcium phosphate, and colloidal silicon dioxide were added to a mixing vessel and mixed for 15 min. Magnesium stearate was then added, and the materials were mixed for 10 min. The mixture was then tableted using a rotary tablet press to give nebivolol-amlodipine plain tablets.

Step 3: The nebivolol-amlodipine plain tablets obtained in step 2 were coated with an aqueous solution of a film coating premix (12% solid content; the percentage refers to the percentage of the mass of the coating premix in the total mass of the aqueous solution of the coating premix) to give nebivolol-amlodipine tablets.

### Example 3

### 1) Formula composition

| Name | Amount per tablet (mg) | Percent (%) |
|---|---|---|
| **Amlodipine layer** | | |
| Amlodipine besylate | 6.93 | 6.93 |
| Microcrystalline cellulose 102 | 60 | 60.00 |
| Lactose monohydrate | 28.07 | 28.07 |
| Sodium carboxymethyl starch | 4 | 4.00 |
| Magnesium stearate | 1 | 1.00 |

| **Nebivolol layer** | | |
|---|---|---|
| Nebivolol hydrochloride | 5.45 | 2.73 |
| Microcrystalline cellulose 101 | 127.55 | 63.77 |
| Lactose monohydrate | 30 | 15.00 |
| Croscarmellose sodium | 12 | 6.00 |
| Pregelatinized starch | 16 | 8.00 |
| Polysorbate-80 | 6 | 3.00 |
| Colloidal silicon dioxide | 1 | 0.50 |
| Magnesium stearate | 2 | 1.00 |

| **Coating material** | | |
|---|---|---|
| Film coating premix | 12 | / |

### 2) Preparation method

### Step A: Nebivolol layer final mixed material preparation:

Pregelatinized starch was slowly added to purified water being stirred, and dissolved, and polysorbate-80 was then added. The mixture was stirred homogeneously and set aside for later use.

Nebivolol hydrochloride, microcrystalline cellulose 101, lactose monohydrate, and croscarmellose sodium were added to a wet granulator. The stirring and cutting functions were activated, and the materials were mixed for 10 min. The aqueous adhesive solution was added, and slurry addition and granulation were performed. The granulated soft material was passed through a 16-mesh sieve for wet sizing. The wet granules were then placed into a thermostatic drying oven and dried until the water content was ≤3.0%. The dried granules were dry-sized using a 20-mesh sieve. Subsequently, the dry granules of nebivolol, colloidal silicon dioxide, and magnesium stearate were added to a three-dimensional mixer and mixed for 5 min to give final mixed granules of nebivolol.

### Step B: Amlodipine layer final mixed material preparation:

Amlodipine besylate, microcrystalline cellulose 102, lactose monohydrate, and sodium carboxymethylcellulose were placed into a three-dimensional mixer and mixed for 20 min. A dry granulator was used; the rolling pressure, the rolling speed, and the feeding rate were adjusted for dry granulation. The resulting granules were dry-sized using a 24-mesh sieve. Magnesium stearate was then added, and the materials were mixed for 5 min to give final mixed granules of amlodipine.

Step C: The final mixed granules of nebivolol obtained in step A and the final mixed granules of amlodipine obtained in step B were tableted using a double-layer tablet press to give nebivolol-amlodipine plain tablets, and film coating was then performed to give nebivolol-amlodipine tablets.

### Example 4

### 1) Formula composition

| Name | Amount per tablet (mg) | Percent (%) |
|---|---|---|
| **Amlodipine layer** | | |
| Amlodipine besylate | 13.86 | 13.86 |
| Microcrystalline cellulose 102 | 54.14 | 54.14 |
| Anhydrous dicalcium phosphate | 25 | 25.00 |
| Sodium carboxymethyl starch | 6 | 6.00 |
| Magnesium stearate | 1 | 1.00 |
| **Nebivolol layer** | | |
| Nebivolol hydrochloride | 10.9 | 5.45 |
| Microcrystalline cellulose 101 | 112.1 | 56.05 |
| Mannitol | 50 | 25.00 |
| Croscarmellose sodium | 10 | 5.00 |
| Hydroxypropylcellulose | 10 | 5.00 |
| Polysorbate-80 | 4 | 2.00 |
| Colloidal silicon dioxide | 1 | 0.50 |
| Magnesium stearate | 2 | 1.00 |
| **Coating material** | | |
| Film coating premix | 12 | / |

### 2) Preparation method

### Amlodipine layer final mixed material preparation:

Amlodipine besylate, microcrystalline cellulose 102, lactose monohydrate, microcrystalline cellulose 102, and sodium carboxymethyl starch were placed into a three-dimensional mixer and mixed for 20 min. Magnesium stearate was then added, and the materials were mixed for 5 min.

### Nebivolol layer final mixed material preparation:

Hydroxypropyl methylcellulose was slowly added to purified water being stirred, and dissolved, and polysorbate-80 was then added. The mixture was stirred homogeneously and set aside for later use.

Nebivolol hydrochloride, microcrystalline cellulose 101, mannitol, and croscarmellose sodium were added to a three-dimensional mixer and mixed for 10 min. The mixed material was then placed into a fluidized bed, and the inlet airflow rate, inlet air temperature, atomization temperature, and feeding rate parameters were set. The aqueous adhesive solution was added using a peristaltic pump, and fluidized granulation was performed. The granulated material was passed through a 30-mesh sieve to give dry granules of nebivolol. Magnesium stearate and colloidal silicon dioxide were then added, and the materials were mixed for 5 min to give final mixed granules of nebivolol.

The final mixed material of amlodipine and the final mixed granules of nebivolol were tableted using a double-layer tablet press to give nebivolol-amlodipine plain tablets, and film coating was then performed to give nebivolol-amlodipine tablets.

### Example 5

### 1) Formula composition

| Name | Amount per tablet (mg) | Percent (%) |
|---|---|---|
| **Internal** | | |
| Nebivolol hydrochloride | 5.45 | 2.27 |
| Lactose monohydrate | 142.59 | 59.41 |
| Pregelatinized starch | 46 | 19.16 |
| Croscarmellose sodium (internal) | 6.9 | 2.87 |

| **Adhesive** | | |
|---|---|---|
| Hydroxypropyl methylcellulose | 3.91 | 1.63 |
| Polysorbate-80 | 0.9 | 0.38 |
| Sodium dodecyl sulfate | 0.6 | 0.25 |
| Purified water | 96 | / |

| **External** | | |
|---|---|---|
| Amlodipine besylate | 6.93 | 2.89 |
| Croscarmellose sodium (external) | 8.97 | 3.74 |
| Colloidal silicon dioxide | 0.6 | 0.25 |
| Microcrystalline cellulose 101 | 15.95 | 6.65 |
| Magnesium stearate | 1.2 | 0.50 |

### 2) Preparation method

Adhesive preparation: An adhesive was prepared from hydroxypropyl methylcellulose and purified water, and polysorbate-80 and sodium dodecyl sulfate were then added. The mixture was stirred homogeneously and set aside for later use.

Step 1: Nebivolol hydrochloride, lactose monohydrate, pregelatinized starch, and croscarmellose sodium (internal) were added to a mixing vessel and mixed for 20 min. The mixed material was then placed into a fluidized bed, and the inlet airflow rate, inlet air temperature, atomization temperature, and feeding rate parameters were set. The aqueous adhesive solution was added using a peristaltic pump, and fluidized granulation was performed. The granulated material was dry-sized using a sizing machine to give dry granules of nebivolol.

Step 2: The dry granules of nebivolol obtained in step 1, amlodipine besylate, croscarmellose sodium (external), colloidal silicon dioxide, and microcrystalline cellulose 101 were added to a mixing vessel and mixed for 10 min, and sizing was then performed using a sizing machine. After the sizing was completed, the material was mixed for another 10 min. Magnesium stearate was added, and the materials were mixed for 5 min. The mixture was then tableted using a rotary tablet press.

### Example 6

### 1) Formula composition

| Name | Amount per tablet (mg) | Percent (%) |
|---|---|---|
| **Internal** | | |
| Nebivolol hydrochloride | 5.45 | 2.27 |
| Lactose monohydrate | 142.44 | 59.35 |
| Pregelatinized starch | 46 | 19.16 |
| Croscarmellose sodium (internal) | 6.9 | 2.87 |

| **Adhesive** | | |
|---|---|---|
| Hydroxypropyl methylcellulose | 3.91 | 1.63 |
| Polysorbate-80 | 0.45 | 0.19 |
| Purified water | 96 | / |

| **External** | | |
|---|---|---|
| Amlodipine besylate | 6.93 | 2.89 |
| Croscarmellose sodium (external) | 8.97 | 3.74 |
| Colloidal silicon dioxide | 0.6 | 0.25 |
| Microcrystalline cellulose 101 | 15.95 | 6.65 |
| Sodium dodecyl sulfate | 1.2 | 0.50 |
| Magnesium stearate | 1.2 | 0.50 |

### 2) Preparation method

Adhesive preparation: An adhesive was prepared from hydroxypropyl methylcellulose and purified water, and polysorbate-80 was then added. The mixture was stirred homogeneously and set aside for later use.

Step 1: Nebivolol hydrochloride, lactose monohydrate, pregelatinized starch, and croscarmellose sodium (internal) were added to a mixing vessel and mixed for 20 min. The mixed material was then placed into a fluidized bed, and the inlet airflow rate, inlet air temperature, atomization temperature, and feeding rate parameters were set. The aqueous adhesive solution was added using a peristaltic pump, and fluidized granulation was performed. The granulated material was sized using a 20-mesh sieve to give dry granules of nebivolol.

Step 2: The dry granules of nebivolol obtained in step 1, amlodipine besylate, croscarmellose sodium (external), colloidal silicon dioxide, microcrystalline cellulose 101, and sodium dodecyl sulfate were added to a mixing vessel and mixed for 10 min. Magnesium stearate was then added, and the materials were mixed for 5 min. The mixture was then tableted using a rotary tablet press.

### Example 7

### 1) Formula composition

| Name | Amount per tablet (mg) | Percent (%) |
|---|---|---|
| **Internal** | | |
| Nebivolol hydrochloride | 5.45 | 2.27 |
| Lactose monohydrate | 141.99 | 59.16 |
| Pregelatinized starch | 46 | 19.16 |
| Croscarmellose sodium (internal) | 6.9 | 2.87 |

| **Adhesive** | | |
|---|---|---|
| Hydroxypropyl methylcellulose | 3.91 | 1.63 |
| Polysorbate-80 | 0.9 | 0.38 |
| Sodium dodecyl sulfate | 1.2 | 0.50 |
| Purified water | 96 | / |

| **External** | | |
|---|---|---|
| Amlodipine besylate | 6.93 | 2.89 |
| Croscarmellose sodium (external) | 8.97 | 3.74 |
| Colloidal silicon dioxide | 0.6 | 0.25 |
| Microcrystalline cellulose 101 | 15.95 | 6.65 |
| Magnesium stearate | 1.2 | 0.50 |

### 2) Preparation method

Adhesive preparation: An adhesive was prepared from hydroxypropyl methylcellulose and purified water, and polysorbate-80 and sodium dodecyl sulfate were then added. The mixture was stirred homogeneously and set aside for later use.

Step 1: Nebivolol hydrochloride, lactose monohydrate, pregelatinized starch, and croscarmellose sodium (internal) were added to a mixing vessel and mixed for 20 min. The mixed material was then placed into a fluidized bed, and the inlet airflow rate, inlet air temperature, atomization temperature, and feeding rate parameters were set. The aqueous adhesive solution was added using a peristaltic pump, and fluidized granulation was performed. The granulated material was dry-sized using a sizing machine to give dry granules of nebivolol.

Step 2: The dry granules of nebivolol obtained in step 1, amlodipine besylate, croscarmellose sodium (external), colloidal silicon dioxide, and microcrystalline cellulose 101 were added to a mixing vessel and mixed for 10 min, and sizing was then performed using a sizing machine. After the sizing was completed, the material was mixed for another 10 min. Magnesium stearate was added, and the materials were mixed for 5 min. The mixture was then tableted using a rotary tablet press.

### Example 8

### 1) Formula composition

| Name | Amount per tablet (mg) | Percent (%) |
|---|---|---|
| **Amlodipine granules** | | |
| Amlodipine besylate | 6.93 | 1.98 |
| Microcrystalline cellulose (internal) | 80 | 22.85 |
| Anhydrous dicalcium phosphate | 40 | 11.43 |

| **Nebivolol granules** | | |
|---|---|---|
| Nebivolol hydrochloride | 5.45 | 1.56 |
| Lactose monohydrate | 133.87 | 38.25 |
| Pregelatinized starch | 40 | 11.43 |
| Hydroxypropyl methylcellulose | 4.5 | 1.29 |
| Polysorbate-80 | 0.9 | 0.26 |
| Sodium dodecyl sulfate | 0.6 | 0.17 |
| Purified water | q.s. | / |

| **External materials** | | |
|---|---|---|
| Microcrystalline cellulose (external) | 15 | 4.28 |
| Croscarmellose sodium (external) | 17.5 | 5.00 |
| Colloidal silicon dioxide | 1.75 | 0.50 |
| Magnesium stearate | 3.5 | 1.00 |
| Total weight of granules | 350 | 100.00 |

### 2) Preparation method

### Amlodipine granule preparation:

Amlodipine besylate, microcrystalline cellulose (internal), and anhydrous dicalcium phosphate were placed in a mixer and mixed for 20 min. A dry granulator was used; the rolling and feeding parameters were adjusted for granulation. The granules were then sized using a 24-mesh sieve to give amlodipine granules.

### Nebivolol granule preparation:

Adhesive solution preparation: Hydroxypropyl methylcellulose was slowly added to purified water being stirred, and dissolved, and sodium dodecyl sulfate and polysorbate-80 were then added and dissolved by stirring. The resulting solution was set aside for later use.

Nebivolol hydrochloride, lactose monohydrate, and pregelatinized starch were added to a three-dimensional mixer and mixed for 10 min. The mixed material was then placed into a fluidized bed, and the inlet airflow rate, inlet air temperature, atomization temperature, and feeding rate parameters were set. The aqueous adhesive solution was added using a peristaltic pump, and fluidized granulation was performed. The granulated material was passed through a 30-mesh sieve to give nebivolol granules.

The amlodipine granules, the nebivolol granules, microcrystalline cellulose (external), croscarmellose sodium (external), and colloidal silicon dioxide were added to a mixing vessel and mixed for 10 min. Magnesium stearate was then added, and the materials were mixed for 5 min to give nebivolol-amlodipine final mixed granules. The final mixed granules were capsulated to give nebivolol-amlodipine capsules.

A dissolution experiment of the amlodipine-nebivolol compound formulations of Examples 1-8 was conducted in 900 mL of a pH 4.5 acetate buffer using USP Method Two at a rotation speed of 50 rpm. The strength of the amlodipine-nebivolol compound formulation in Example 4 was 10 mg (amlodipine)/10 mg (nebivolol). The strength of the amlodipine-nebivolol compound formulations in Examples 1-3 and 5-8 was 5 mg (amlodipine)/5 mg (nebivolol). The dissolution results are shown in Table 1.

**Table 1. - Dissolution results for amlodipine-nebivolol compound formulations in a pH 4.5 medium**

| No. | Example 1 | | | |
|---|---|---|---|---|
| Active ingredient | Amlodipine | | Nebivolol | |
| Time (min) | Cumulative dissolution rate (%) | RSD (%) | Cumulative dissolution rate (%) | RSD (%) |
| 5 | 60 | 13.2 | 48 | 10.0 |
| 10 | 82 | 4.5 | 70 | 7.9 |
| 20 | 92 | 3.3 | 86 | 5.1 |
| 30 | 92 | 3.7 | 90 | 4.1 |
| 45 | 93 | 3.5 | 93 | 3.4 |
| 60 | 93 | 3.5 | 96 | 2.2 |

| No. | Example 2 | | | |
|---|---|---|---|---|
| Active ingredient | Amlodipine | | Nebivolol | |
| Time (min) | Cumulative dissolution rate (%) | RSD (%) | Cumulative dissolution rate (%) | RSD (%) |
| 5 | 53 | 13.2 | 51 | 12.9 |
| 10 | 71 | 12.8 | 70 | 13.4 |
| 20 | 88 | 5.8 | 89 | 6.8 |
| 30 | 90 | 5.7 | 92 | 7.5 |
| 45 | 91 | 5.8 | 93 | 7.6 |
| 60 | 92 | 4.1 | 94 | 6.4 |

| No. | Example 3 | | | |
|---|---|---|---|---|
| Active ingredient | Amlodipine | | Nebivolol | |
| Time (min) | Cumulative dissolution rate (%) | RSD (%) | Cumulative dissolution rate (%) | RSD (%) |
| 5 | 40 | 10.8 | 38 | 11.3 |
| 10 | 56 | 8.5 | 54 | 8.9 |
| 20 | 78 | 5.9 | 78 | 6.2 |
| 30 | 83 | 5.4 | 84 | 5.9 |
| 45 | 89 | 4.6 | 90 | 4.3 |
| 60 | 90 | 4.0 | 92 | 3.8 |

| No. | Example 4 | | | |
|---|---|---|---|---|
| Active ingredient | Amlodipine | | Nebivolol | |
| Time (min) | Cumulative dissolution rate (%) | RSD (%) | Cumulative dissolution rate (%) | RSD (%) |
| 5 | 33 | 13.8 | 26 | 15.6 |
| 10 | 52 | 8.9 | 52 | 9.6 |
| 20 | 77 | 6.2 | 76 | 7.5 |
| 30 | 90 | 3.1 | 88 | 6.2 |
| 45 | 93 | 2.6 | 93 | 2.8 |
| 60 | 93 | 2.8 | 95 | 2.4 |

| No. | Example 5 | | | |
|---|---|---|---|---|
| Active ingredient | Amlodipine | | Nebivolol | |
| Time (min) | Cumulative dissolution rate (%) | RSD (%) | Cumulative dissolution rate (%) | RSD (%) |
| 5 | 53 | 11.6 | 47 | 10.8 |
| 10 | 67 | 12.0 | 61 | 11.1 |
| 20 | 83 | 7.1 | 78 | 7.3 |
| 30 | 89 | 6.5 | 86 | 6.3 |
| 45 | 92 | 5.6 | 92 | 6.0 |
| 60 | 95 | 4.7 | 95 | 4.4 |

| No. | Example 6 | | | |
|---|---|---|---|---|
| Active ingredient | Amlodipine | | Nebivolol | |
| Time (min) | Cumulative dissolution rate (%) | RSD (%) | Cumulative dissolution rate (%) | RSD (%) |
| 5 | 60 | 9.8 | 52 | 10.7 |
| 10 | 75 | 7.6 | 66 | 7.4 |
| 20 | 84 | 6.4 | 76 | 6.5 |
| 30 | 93 | 5.2 | 84 | 4.9 |
| 45 | 99 | 3.1 | 90 | 3.0 |
| 60 | 102 | 2.7 | 93 | 3.2 |

| No. | Example 7 | | | |
|---|---|---|---|---|
| Active ingredient | Amlodipine | | Nebivolol | |
| Time (min) | Cumulative dissolution rate (%) | RSD (%) | Cumulative dissolution rate (%) | RSD (%) |
| 5 | 53 | 11.7 | 48 | 10.5 |
| 10 | 76 | 7.3 | 71 | 7.0 |
| 20 | 91 | 5.0 | 87 | 5.4 |
| 30 | 94 | 3.0 | 92 | 2.6 |
| 45 | 95 | 2.0 | 92 | 2.4 |
| 60 | 96 | 2.0 | 93 | 1.6 |

| No. | Example 8 | | | |
|---|---|---|---|---|
| Active ingredient | Amlodipine | | Nebivolol | |
| Time (min) | Cumulative dissolution rate (%) | RSD (%) | Cumulative dissolution rate (%) | RSD (%) |
| 5 | 45 | 8.8 | 39 | 10.2 |
| 10 | 69 | 7.2 | 65 | 8.9 |
| 20 | 86 | 5.6 | 81 | 5.4 |
| 30 | 91 | 3.0 | 88 | 3.2 |
| 45 | 94 | 2.4 | 91 | 2.4 |
| 60 | 97 | 2.1 | 93 | 2.0 |

The compound formulations of nebivolol and amlodipine provided by the present disclosure showed good synergy, which is conducive to enhancing antihypertensive effects, improving safety and tolerability, and reducing side effects. Compared to monotherapy, the combined use of nebivolol and amlodipine has the following advantages: (1) reducing the number of pills that need to be taken and thus improving patient compliance; (2) helping improve adherence for elderly people or people with swallowing difficulties; (3) maintaining stable plasma concentrations *in vivo,* maintaining stable blood pressures in patients with hypertension over the long run, and reducing the side effects of each of the drugs; and (4) preventing patients from interrupting medication at will and preventing disease recurrence and progression of severe complications.

The embodiments of the present disclosure have been described above. However, the present disclosure is not limited to the embodiments described above. Any modification, equivalent replacement, improvement, and the like made without departing from the spirit and principle of the present disclosure shall fall within the protection scope of the present disclosure.

## Claims

1. A nebivolol and amlodipine composition, wherein the nebivolol and amlodipine composition comprises an active ingredient nebivolol and an active ingredient amlodipine, the active ingredient nebivolol comprises one or more of nebivolol, a pharmaceutically acceptable salt thereof, and a solvate thereof, and the active ingredient amlodipine comprises one or more of amlodipine, a pharmaceutically acceptable salt thereof, and a solvate thereof.

2. The nebivolol and amlodipine composition as claimed in claim 1, wherein:
the pharmaceutically acceptable salt of nebivolol is nebivolol hydrochloride;
and/or,
the pharmaceutically acceptable salt of amlodipine is amlodipine besylate and/or amlodipine maleate.

3. The nebivolol and amlodipine composition as claimed in claim 1, wherein:
the nebivolol and amlodipine composition further comprises one or more of a surfactant, a filler, an adhesive, a disintegrant, a lubricant, a glidant, a colorant, and a coating film.

4. The nebivolol and amlodipine composition as claimed in claim 3, wherein:
the surfactant is selected from sodium dodecyl sulfate and/or polysorbate;
and/or,
the filler is selected from one or more of microcrystalline cellulose, dicalcium phosphate, mannitol, sucrose, glucose, maltose, lactose, sorbitol, xylitol, maltitol, galactitol, erythritol, dextrin, and trehalose;
and/or,
the adhesive is selected from one or more of polyoxyethylene, hydroxypropyl methylcellulose, hydroxypropylcellulose, hydroxyethylcellulose, ethylcellulose, copovidone, povidone, pregelatinized starch, acacia, polyvinylpyrrolidone, and sodium alginate;
and/or,
the disintegrant is selected from one or more of crospovidone, croscarmellose sodium, sodium carboxymethyl starch, and corn starch;
and/or,
the lubricant is selected from one or more of a metal stearate, stearic acid, talc, a stearic acid ester, stearyl fumarate, and micronized silica gel;
and/or,
the glidant is selected from one or more of talc, micronized silica gel, and colloidal silicon dioxide;
and/or,
the colorant is selected from iron oxide red and/or iron oxide yellow;
and/or,
the coating film is a film coating premix.

5. The nebivolol and amlodipine composition as claimed in any one of claims 1-4, wherein components in the nebivolol and amlodipine composition, other than the coating film, collectively constitute a core; the core is selected from any one of the following formulas:
formula 1: 2.77% amlodipine besylate, 2.18% nebivolol hydrochloride, 44.25% microcrystalline cellulose 101, 16.00% lactose monohydrate, 12.00% pregelatinized starch, 6.00% sodium carboxymethyl starch, 6.00% hydroxypropylcellulose, 1.20% polysorbate-80, 8.00% microcrystalline cellulose 102, 0.60% colloidal silicon dioxide, and 1.00% magnesium stearate and coating material, wherein each of the percentages refers to a percentage of the corresponding component's mass in the core's total mass;
formula 2:
2.31% amlodipine besylate, 1.82% nebivolol hydrochloride, 39.70% mannitol, 20.00% lactose monohydrate, 6.67% hydroxypropyl methylcellulose, 6.00% croscarmellose sodium, 2.00% sodium dodecyl sulfate, 10.00% microcrystalline cellulose 102, 10.00% anhydrous dicalcium phosphate, 0.50% colloidal silicon dioxide, and 1.00% magnesium stearate and coating material, wherein each of the percentages refers to a percentage of the corresponding component's mass in the core's total mass;
formula 3:
an amlodipine layer: 6.93% amlodipine besylate, 60.00% microcrystalline cellulose 102, 28.07% lactose monohydrate, 4.00% sodium carboxymethyl starch, and 1.00% magnesium stearate, wherein each of the percentages refers to a percentage of the corresponding component's weight in the amlodipine layer's total mass; and
a nebivolol layer: 2.73% nebivolol hydrochloride, 63.77% microcrystalline cellulose 101, 15.00% lactose monohydrate, 6.00% croscarmellose sodium, 8.00% pregelatinized starch, 3.00% polysorbate-80, 0.50% colloidal silicon dioxide, and 1.00% magnesium stearate, wherein each of the percentages refers to a percentage of the corresponding component's weight in the nebivolol layer's total mass;
formula 4:
an amlodipine layer: 13.86% amlodipine besylate, 54.14% microcrystalline cellulose 102, 25.00% anhydrous dicalcium phosphate, 6.00% sodium carboxymethyl starch, and 1.00% magnesium stearate, wherein each of the percentages refers to a percentage of the corresponding component's weight in the amlodipine layer's total mass; and
a nebivolol layer: 5.45% nebivolol hydrochloride, 56.05% microcrystalline cellulose 101, 25.00% mannitol, 5.00% croscarmellose sodium, 5.00% hydroxypropylcellulose, 2.00% polysorbate-80, 0.50% colloidal silicon dioxide, and 1.00% magnesium stearate, wherein each of the percentages refers to a percentage of the corresponding component's weight in the nebivolol layer's total mass;
formula 5: 2.27% nebivolol hydrochloride, 2.89% amlodipine besylate, 59.41% lactose monohydrate, 19.16% pregelatinized starch, 2.87% croscarmellose sodium (internal), 1.63% hydroxypropyl methylcellulose, 0.38% polysorbate-80, and 0.25% sodium dodecyl sulfate, 3.74% croscarmellose sodium (external), 0.25% colloidal silicon dioxide, 6.65% microcrystalline cellulose 101, and 0.50% magnesium stearate, wherein each of the percentages refers to a percentage of the corresponding component's mass in the core's total mass;
formula 6: 2.27% nebivolol hydrochloride, 2.89% amlodipine besylate, 59.35% lactose monohydrate, 19.16% pregelatinized starch, 2.87% croscarmellose sodium (internal), 1.63% hydroxypropyl methylcellulose, 0.19% polysorbate-80, 3.74% croscarmellose sodium (external), 0.25% colloidal silicon dioxide, 6.65% microcrystalline cellulose 101, 0.50% sodium dodecyl sulfate, and 0.50% magnesium stearate, wherein each of the percentages refers to a percentage of the corresponding component's mass in the core's total mass;
formula 7: 2.27% nebivolol hydrochloride, 2.89% amlodipine besylate, 59.16% lactose monohydrate, 19.16% pregelatinized starch, 2.87% croscarmellose sodium (internal), 1.63% hydroxypropyl methylcellulose, 0.38% polysorbate-80, 0.50% sodium dodecyl sulfate, 3.74% croscarmellose sodium (external), 0.25% colloidal silicon dioxide, 6.65% microcrystalline cellulose 101, and 0.50% magnesium stearate, wherein each of the percentages refers to a percentage of the corresponding component's mass in the core's total mass; and
formula 8: 1.56% nebivolol hydrochloride, 1.98% amlodipine besylate, 22.85% microcrystalline cellulose (internal), 11.43% anhydrous dicalcium phosphate, 38.25% lactose monohydrate, 11.43% pregelatinized starch, 1.29% hydroxypropyl methylcellulose, 0.26% polysorbate-80, 0.17% sodium dodecyl sulfate, 4.28% microcrystalline cellulose (external), 5.00% croscarmellose sodium (external), 0.50% colloidal silicon dioxide, and 1.00% magnesium stearate, wherein each of the percentages refers to a percentage of the corresponding component's mass in the core's total mass.

6. Use of the nebivolol and amlodipine composition as claimed in any one of claims 1-5 for manufacturing a medicament for the treatment and/or prevention of cardiovascular diseases.

7. The use as claimed in claim 6, wherein the cardiovascular diseases include hypertension, heart failure, coronary heart disease, angina, arrhythmia, myocardial infarction, congenital heart disease, and heart valve disease.

8. Use of the nebivolol and amlodipine composition as claimed in any one of claims 1-5 for manufacturing a pharmaceutical formulation, wherein the pharmaceutical formulation is preferably an oral pharmaceutical formulation.

9. The use as claimed in claim 8, wherein dosage forms of the oral pharmaceutical formulation include, but are not limited to, compound tablet, double-layer tablet, capsule, pellet, and micro-tablet.

10. A preparation method for the nebivolol and amlodipine composition as claimed in any one of claims 1-5, comprising, but not limited to, a direct powder compression method, a wet granulation method, a dry granulation method, a fluidized granulation method, an extrusion-spheronization method, and a pellet coating method.
